# EUROPÄISCHE PATENTSCHRIFT

(11) **EP 1 866 287 B1**
(45) Veröffentlichungstag und Bekanntmachung des Hinweises auf die Patenterteilung: **21.09.2016**
(21) Anmeldenummer: 06707604.2
(22) Anmeldetag: 18.03.2006
(51) Int. Cl.: C07D 231/20, C07D 401/12, C07D 409/12, A01N 43/56

(54) **SUBSTITUIERTE PYRAZOLYLOXYPHENYLDERIVATE ALS HERBIZIDE**
SUBSTITUTED PYRAZOLYL OXYPHENYL DERIVATIVES USED AS HERBICIDES
DERIVES DE PYRAZOLYLOXYPHENYLE SUBSTITUES EN TANT QU'HERBICIDES

(30) Priorität: 31.03.2005 DE 102005014638
(43) Veröffentlichungstag der Anmeldung: 19.12.2007
(73) Patentinhaber: Bayer Intellectual Property GmbH, 40789 Monheim am Rhein (DE)
(72) Erfinder: HELMKE, Hendrik, 65835 Liederbach (DE); HOFFMANN, Michael, Gerhard, 65439 Flörsheim (DE); WILLMS, Lothar, 65719 Hofheim (DE); AULER, Thomas, 42799 Leichlingen (DE); KEHNE, Heinz, 65719 Hofheim (DE); HILLS, Martin, 65510 Idstein (DE); FEUCHT, Dieter, 65760 Eschborn (DE)
(74) Vertreter: BIP Patents
(86) Internationale Anmeldenummer: PCT/EP2006/002507
(87) Internationale Veröffentlichungsnummer: WO 2006/103003

(56) Entgegenhaltungen:
- WO-A-94/22833
- WO-A-03/051846

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus US 5698495, WO 9718196 ist bereits bekannt, daß bestimmte Pyrazolyloxyphenylderivate herbizide Eigenschaften besitzen. WO 2003/051846 beschreibt ebenfalls Pyrazolyloxyphenylderivate, die einen substituierten und über ein Sauerstoffatom gebundenen Rest aus der Gruppe Phenyl, Pyridyl, Pyrazolyl und Thienyl tragen. WO 1994/22833 offenbart herbizid wirksame 2,6-[Di(hetero)aryloxy]-pyridinderivate, die in 4-Position unsubstituiert oder durch Methylgruppe substituiert sind.

Die aus diesen Schriften bekannten Verbindungen zeigen jedoch häufig eine nicht ausreichende herbizide Wirksamkeit. Aufgabe der vorliegenden Erfindung ist daher die Bereitstellung von herbizid wirksamen Verbindungen mit - gegenüber den aus dem Stand der Technik bekannten Verbindungen - verbesserten herbiziden Eigenschaften.

Es wurde nun gefunden, daß Pyrazolyloxyphenylderivate, die durch ausgewählte Reste substituiert sind, als Herbizide besonders gut geeignet sind. Ein Gegenstand der vorliegenden Erfindung sind daher Verbindungen der Formel (I) und deren Salze, 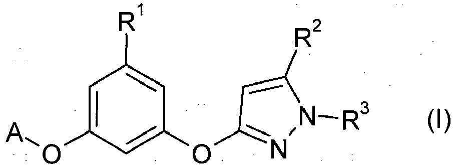 worin die Substituenten und Indices folgende Bedeutungen haben:
- R¹: bedeutet Brom, Chlor, Fluor oder Iod;
- R²: bedeutet Trifluormethyl, Difluormethyl oder Chlordifluormethyl
- R³: bedeutet Methyl oder Ethyl;
- A: bedeutet einen Rest aus der Gruppe umfassend die Reste A1 bis A4

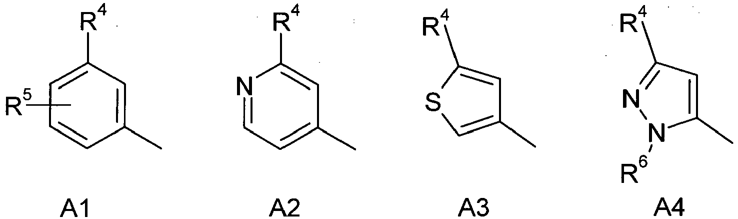

- R⁴: bedeutet Fluormethyl, Difluormethyl, Trifluormethyl, Fluormethoxy, Difluormethoxy, Trifluormethyoxy, Chlor oder Cyano;
- R⁵: bedeutet Wasserstoff, (C₁-C₈)-Alkyl, Brom, Chlor, Fluor, Iod oder Cyano, und
- R⁶: bedeutet (C₁-C₈)-Alkyl.

Je nach Art der Substituenten können die Verbindungen der allgemeinen Formel (I) eine Säure anlagern, beispielsweise Salzsäure. Die dadurch entstehenden Säureaddukte, wie Hydrochloride, sind ebenfalls Gegenstand der Erfindung.

In Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Bevorzugt sind Verbindungen der allgemeinen Formel (I), worin
- R¹: bedeutet Brom, Chlor, Fluor oder Iod;
- R²: bedeutet Trifluormethyl oder Difluormethyl;
- R³: bedeutet Methyl oder Ethyl;
- A: bedeutet einen Rest aus der Gruppe umfassend die Reste A1 bis A4;
- R⁴: bedeutet Difluormethyl,Trifluormethyl, Difluormethoxy, Trifluormethoxy, Chlor oder Cyano;
- R⁵: bedeutet Wasserstoff, Fluor oder Chlor;
- R⁶: bedeutet Methyl oder Ethyl.

Besonders bevorzugt sind Verbindungen der allgemeinen Formel (I), worin
- R¹: bedeutet Brom, Chlor, Fluor oder Iod;
- R²: bedeutet Trifluormethyl oder Difluormethyl;
- R³: bedeutet Methyl oder Ethyl;
- A: bedeutet einen Rest aus der Gruppe umfassend die Reste A1 bis A4;
- R⁴: bedeutet Difluormethyl, Trifluormethyl, Difluormethoxy, Trifluormethoxy, Chlor oder Cyano;
- R⁵: bedeutet Wasserstoff oder Fluor;
- R⁶: bedeutet Methyl.

Ganz besonders bevorzugt sind Verbindungen der allgemeinen Formel (I), worin
- R¹: bedeutet Brom, Chlor, Fluor oder Iod;
- R²: bedeutet Trifluormethyl oder Difluormethyl;
- R³: bedeutet Methyl oder Ethyl;
- A: bedeutet den Rest A1;
- R⁴: bedeutet Difluormethyl, Trifluormethyl, Difluormethoxy, Trifluormethoxy, Chlor oder Cyano;
- R⁵: bedeutet Wasserstoff oder Fluor.

Ebenfalls ganz besonders bevorzugt sind Verbindungen der allgemeinen Formel (I), worin
- R¹: bedeutet Brom, Chlor, Fluor oder Iod;
- R²: bedeutet Trifluormethyl oder Difluormethyl;
- R³: bedeutet Methyl oder Ethyl;
- A: bedeutet den Rest A2;
- R⁴: bedeutet Difluormethyl, Trifluormethyl, Difluormethoxy, Trifluormethoxy, Chlor oder Cyano.

Darüberhinaus ganz besonders bevorzugt sind Verbindungen der allgemeinen Formel (I), worin
- R¹: bedeutet Brom, Chlor, Fluor oder Iod;
- R²: bedeutet Trifluormethyl oder Difluormethyl;
- R³: bedeutet Methyl oder Ethyl;
- A: bedeutet den Rest A3;
- R⁴: bedeutet Difluormethyl, Trifluormethyl, Difluormethoxy, Trifluormethoxy, Chlor oder Cyano.

Ebenso ganz besonders bevorzugt sind Verbindungen der allgemeinen Formel (I), worin
- R¹: bedeutet Brom, Chlor, Fluor oder Iod;
- R²: bedeutet Trifluormethyl oder Difluormethyl;
- R³: bedeutet Methyl oder Ethyl;
- A: bedeutet den Rest A4;
- R⁴: bedeutet Difluormethyl, Trifluormethyl, Difluormethoxy, Trifluormethoxy, Chlor oder Cyano;
- R⁶: bedeutet Methyl.

In allen nachfolgend genannten Formeln haben die Substituenten und Symbole, sofern nicht anders definiert, dieselbe Bedeutung wie unter Formel (I) beschrieben.

Erfindungsgemäße Verbindungen der Formel (I) können beispielsweise nach dem in Schema 1 angegebenen Verfahren hergestellt werden. Dabei wird in einem ersten Schritt die Verbindung der Formel (II) baseninduziert mit einer Verbindung A-OH und dann in einem zweiten Schritt ebenfalls baseninduziert mit einer Verbindung (IIIa) zu erfindungsgemäßen Verbindungen der Formel (la), worin R¹ für Nitro steht, umgesetzt. In Verbindungen der Formeln (II) und (IIa) steht LG jeweils für eine Abgangsgruppe, wie Chlor, Fluor oder Pseudohalogen. Diese Reaktionen sind dem Fachmann bekannt.

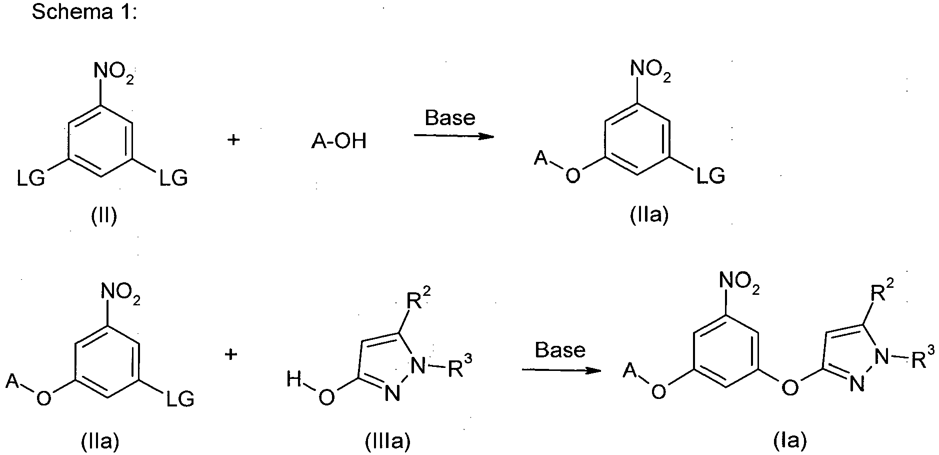

Im Bedarfsfall können die beiden vorstehend genannten Reaktionsschritte auch in umgekehrter Reihenfolge durchgeführt werden.

Erfindungsgemäße Verbindungen der Formel (la) können gemäß Schema 2 zu erfindungsgemäßen Verbindungen der Formel (Ib), worin R¹ für Amino steht, umgesetzt werden. Diese Reaktionen sind dem Fachmann beispielsweise aus R.L. Augustine "Catalytic Hydrogenation" Marcel Dekker, New York 1965, Chpt5 und P.N. Rylander "Hydrogenation Methods" Academic Press, New York 1985, Chpt 8, bekannt.

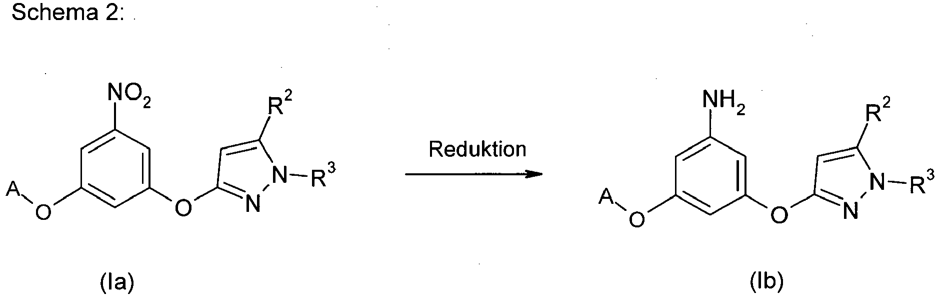

Erfindungsgemäße Verbindungen der Formel (Ic), worin R^{1a} für Brom, Chlor, Fluor oder Iod steht, können gemäß Schema 3 durch Diazotierung und anschließender Funktionalisierung aus den Verbindungen (Ib) hergestellt werden. Die Diazotierung des Anilinderivates (III) und Funktionalisierung der Diazoniumsalze (Verkochung und Reduktion, Schiemann-Reaktion, Balz-Schiemann-Reaktion, Sandmeyer-Reaktionen) sind dem Fachmann bekannt und können nach bekannten Verfahren erfolgen, siehe z.B.
a) F.A. Carey, R.J. Sundberg, "Organische Chemie (Deutsche Ausgabe)" VCH Verlagsgesellschaft, Weinheim 1995, Chpt 24.2.1 und dort zitierte Literatur.
b) Organikum, VEB Deutscher Verlag der Wissenschaften, Berlin 1988, Chpt D.8.2.1, D.8.3.1, D.8.3.2 und unter D.8.6. genannte Literaturhinweise.
c) Schank K., Aromatic diazonium salts. Method. Chim. (1975), 6 159-203.
d) Yoneda, Norihiko; Fukuhara, Tsuyoshi. Preparation of fluoro aromatics. Diazotization, fluorodediazoniation of amino aromatics. Yuki Gosei Kagaku Kyokaishi (1989), 47(7), 619-28.
e) Nonhebel, Derek C. Copper-catalyzed single-electron oxidations and reductions. Special Publication - Chemical Society (1970), No. 24 409-37.

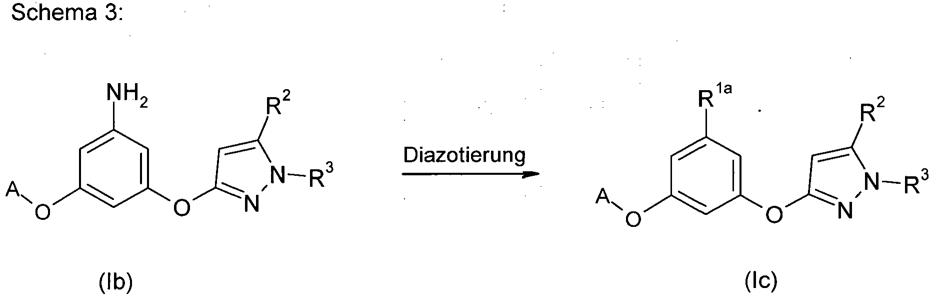

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Apera spica venti, Chenopodium album, Lamium purpureum, Polygonum convulvulus, Stellaria media, Veronica hederifolia, Veronica persica und Viola tricolor.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Insbesondere weisen sie eine ausgezeichnete Verträglichkeit in Getreide, wie Weizen, Gerste und Mais, insbesondere Weizen, auf. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codierenden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemischphysikalischen Parameter vor-gegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapsel-suspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstoffformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder"The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; aminopyralid; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; pinoxaden; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyraclonil, pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1 H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127; KIH-2023 und KIH-485.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

1. Herstellung von 3-Fluor-5-(1-methyl-3-trifluormethylpyrazol-5-yloxy)-nitrobenzol 30.00 g (189 mmol) 3,5-Difluornitrobenzol wurden unter Stickstoffatmosphäre in 150 ml N,N-Dimethylformamid vorgelegt und bei Raumtemperatur (RT) mit 28.67 g (207 mmol) K₂CO₃ und 31.32 g (189 mmol) 1-Methyl-3-(trifluormethyl)-pyrazol-5-on versetzt. Man erwärmte für 33 h auf 85°C und weitere 3 h auf 100°C, kühlte auf RT ab und gab Wasser zur Reaktionslösung hinzu. Man extrahierte dreimal mit Essigester. Die vereinigten Phasen wurden mit Wasser gewaschen und dann über MgSO₄ getrocknet, filtriert und eingeengt. Säulenchromatographie des Rohproduktes ergaben 9.00 g 3-Fluor-5-(1-Methyl-3-trifluormethylpyrazol-5-yloxy)-nitrobenzol in Form eines orange-roten Öls.
   - ¹H-NMR:: δ [CDCl₃] 3.84 ppm (s, 3H), 6.07 (s, 1 H), 7.20 (dt, 1 H), 7.80 (m, 2H)
2. Herstellung von 3-(1-Methyl-5-trifluormethylpyrazol-3-yloxy)-5-(3-trifluormethylphenyloxy)-nitrobenzol 3.64 g (22.0 mmol) 1-Methyl-5-(trifluormethyl)-pyrazol-3-on wurden unter Stickstoffatmosphäre in 100 ml Dimethylacetamid vorgelegt und bei 0°C mit 0.598 g (25.0 mmol) NaH (80%ig) versetzt. Man ließ auf RT kommen und setzte 6.00 g (20.0 mmol) 3-Fluor-5-(3-trifluormethylphenyloxy)-nitrobenzol zu und erwärmte für 2 h auf 90°C und weitere 8 h auf 130°C, kühlte auf RT ab und gab Wasser zur Reaktionslösung und rührte einige Minuten nach. Man extrahierte zweimal mit Heptan/Essigester (1:1) und zweimal mit Essigester. Die vereinigten Phasen wurden mit Wasser gewaschen und anschießend über MgSO₄ getrocknet, filtriert und eingeengt. Säulenchromatographie des Rohproduktes ergaben 4.24 g 3-(1-Methyl-5-trifluormethylpyrazol-3-yloxy)-5-(3-trifluormethylphenyloxy)-nitrobenzol in Form wachsartiger Kristalle.
   - ¹H-NMR:: δ [CDCl₃] 3.92 ppm (s, 3H), 6.28 (s, 1H), 7.14 (t, 1 H), 7.24 (dt, 1 H), 7.34 (s, 1 H), 7.45-7.60 (m, 2H), 7.75 (t, 1 H).
3. Herstellung von 3-(1-Methyl-5-trifluormethylpyrazol-3-yloxy)-5-(3-trifluormethylphenyloxy)-anilin
   4.00 g (9.0 mmol) 3-(1-Methyl-5-trifluormethylpyrazol-3-yloxy)-5-(3-trifluormethylphenyloxy)-nitrobenzol, 5.00 g (45.0 mmol) Ammoniumformiat und 0.50 g (4.0 mmol) Pd(OH)₂ auf Kohlenstoff (20%ig) wurden unter Stickstoffatmosphäre in 100 ml Methanol vorgelegt und für 90 min auf 70°C erwämt. Man kühlte auf RT ab, filtrierte die Reaktionslösung, engte ein und erhielt 3.75 g 3-(1-Methyl-5-trifluormethylpyrazol-3-yloxy)-5-(3-trifluormethylphenyloxy)-anilin als gelbes Öl.
   - ¹H-NMR:: δ [CDCl₃] 3.88 ppm (s, 3H), 6.08 (t, 1H), 6.15 (t, 1H), 6.17 (s, 1H), 6.23, (t, 1H), 7.20 (d, 1H), 7.26 (s, 1H), 7.34 (d, 1H), 7.43 (t, 1H).
4. Herstellung von 1-(1-Methyl-5-trifluormethylpyrazol-3-yloxy)-3-(3-trifluormethylphenyloxy)-benzol
   0.215 g 3-(1-Methyl-5-trifluormethylpyrazol-3-yloxy)-5-(3-trifluormethylphenyloxy)-anilin und 0.159 g (2.0 mmol) n-Butylnitrit wurden unter Stickstoffatmosphäre in 5 ml THF vorgelegt und für 3-4 h auf 40°C erwämt. Man kühlte auf RT ab, gab Wasser zur Reaktionslösung und rührte einige Minuten nach und extrahierte zweimal mit Essigester. Die vereinigten Phasen wurden mit Wasser gewaschen und anschließend über MgSO₄ getrocknet, filtriert und eingeengt. Säulenchromatographie des Rohproduktes ergaben 60 mg 1-(1-Methyl-5-trifluormethylpyrazol-3-yloxy)-3-(3-trifluormethylphenyloxy)-benzol als gelbes Öl.
   - ¹H-NMR:: δ [CDCl₃] 3.85 ppm (s, 3H), 6.08 (s, 1H), 6.77 (dt, 1H), 6.80 (t, 1H), 6.90, (dt, 1H), 7.20 (dt, 1H), 7.28-7.38 (m, 3H), 7.43 (t, 1H).
5. Herstellung von 3-(1-Methyl-5-trifluormethylpyrazol-3-yloxy)-5-(3-trifluormethylphenyloxy)-fluorbenzol
   Zu 0.220 g 3-(1-Methyl-5-trifluormethylpyrazol-3-yloxy)-5-(3-trifluormethylphenyloxy)-anilin gelöst in 5 ml Chlorbenzol wurden unter Schutzgas bei 0°C 0.185 g (2.0 mmol) Nitrosyltetrafluoroborat zugegeben, man rührte 30 min bei RT und erwärmte für 3 h auf 90°C. Man kühlte auf RT ab, gab Wasser dazu, rührte einige Minuten nach und extrahierte zweimal mit Essigester. Die vereinigten Phasen wurden mit Wasser gewaschen und anschließend über MgSO₄ getrocknet, filtriert und eingeengt. Säulenchromatographie des Rohproduktes ergaben 40 mg 3-(1-Methyl-5-trifluormethylpyrazol-3-yloxy)-5-(3-trifluormethylphenyloxy)-fluorbenzol als gelbes Öl. ¹H-NMR: δ [CDCl₃] 3.85 ppm (s, 3H), 6.21 (s, 1H), 6.43 (dt, 1H), 6.58 (s, 1H), 6.63, (dt, 1H), 7.22 (dt, 1H), .7.30 (s, 1H), 7.38-7.50 (bm, 2H).
6. Herstellung von 3-(1-Methyl-5-trifluormethylpyrazol-3-yloxy)-5-(1-methyl-3-trifluormethylpyrazol-5-yloxy)-fluorbenzol
   126 mg 1-Methyl-5-(trifluormethyl)-pyrazol-3-on wurden unter Schutzgas in 5 ml Dimethylacetamid vorgelegt und bei 0°C mit 27 mg NaH (80%ig) versetzt. Man ließ auf RT kommen und setzte 200 mg 5-(1-Methyl-3-trifluormethylpyrazol-5-yloxy)-1,3-difluorbenzol sowie katalytische Mengen Kupferiodid zu und erwärmte für 6 h auf 150°C, kühlte auf RT ab, gab Wasser dazu und rührte einige Minuten nach. Man extrahierte dreimal mit Essigester. Die vereinigten Phasen wurden mit Wasser gewaschen, über MgSO₄ getrocknet, filtriert und eingeengt. Säulenchromatographie des Rohproduktes ergaben 200 mg 3-(1-Methyl-5-trifluormethylpyrazol-3-yloxy)-5-(1-Methyl-3-trifluormethylpyrazol-5-yloxy)-fluorbenzol als gelbes Öl.
   - ¹H-NMR:: δ [CDCl₃] 3.78 ppm (s, 3H), 3.91 (s, 3H), 6.01 (s, 1H), 6.24 (s, 1H), 6.58 (dt, 1H), 6.70 (m, 2H).
7. Herstellung von 3-(1-Methyl-5-trifluormethylpyrazol-3-yloxy)-5-(3-trifluormethylphenyloxy)-chlorbenzol
   0.400 g 3-(1-Methyl-5-trifluormethylpyrazol-3-yloxy)-5-(3-trifluormethylphenyloxy)-anilin wurden in 5 ml Methylenchlorid gelöst und bei 0-5°C mit 0.569 g (6.0 mmol) Kupfer(I)chlorid und anschließend mit 0.593 g (6.0 mmol) n-Butylnitrit versetzt und für 2-3 h bei 0-5°C gerührt. Man ließ über Nacht auf RT kommen, gab Wasser zur Reaktionslösung und rührte einige Minuten nach und extrahierte zweimal mit Methylenchlorid. Die vereinigten Phasen wurden mit Wasser gewaschen und anschießend über MgSO₄ getrocknet, filtriert und eingeengt. Säulenchromatographie des Rohproduktes ergaben 60 mg 1-(1-Methyl-5-trifluormethylpyrazol-3-yloxy)-3-(3-trifluormethylphenyloxy)-chlorbenzol als oranges Öl.
   - ¹H-NMR:: δ [CDCl₃] 3.84 ppm (s, 3H), 6.18 (s, 1H), 6.80 (m, 2H), 6.91 (dt, 1H), 7.18 (dt, 1H), 7.28-7.50 (m, 3H).

Die in nachfolgenden Tabellen aufgeführten Beispiele wurden analog oben genannten Methoden hergestellt beziehungsweise sind analog oben genannten Methoden erhältlich.

Die verwendeten Abkürzungen bedeuten
Et = Ethyl Me = Methyl

**Tabelle A: Erfindungsgemäße Verbindungen der allgemeinen Formel (I), worin die Substituenten und Symbole folgende Bedeutungen haben:**

| | | | | | |
|---|---|---|---|---|---|
| | A = A1 | | | R⁴ = CF₃ | |
| 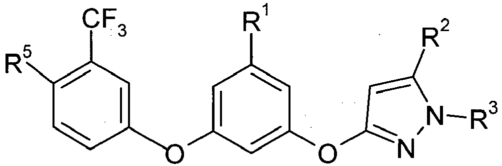 | | | | | |

| **Nr.** | **R¹** | **R²** | **R³** | **R⁵** | **Physikalische Daten: ¹H-NMR (CDCl₃)** |
|---|---|---|---|---|---|
| 1 | H | CF₃ | Me | H | 6.18 (s, Pyrazolyl-H) |
| 2 | F | CF₃ | Me | H | 6.21 (s, Pyrazolyl-H) |
| 3 | Cl | CF₃ | Me | H | 6.21 (s, Pyrazolyl-H) |
| 4 | Br | CF₃ | Me | H | 6.21 (s, Pyrazolyl-H) |
| 5 | I | CF₃ | Me | H | 6.20 (s, 1H, Pyrazolyl-H) |
| 6 | H | CF₃ | Et | H | |
| 7 | F | CF₃ | Et | H | |
| 8 | Cl | CF₃ | Et | H | |
| 9 | Br | CF₃ | Et | H | |
| 10 | I | CF₃ | Et | H | |
| 11 | H | CHF₂ | Me | H | |
| 12 | F | CHF₂ | Me | .H | |
| 13 | Cl | CHF₂ | Me | H | |
| 14 | Br | CHF₂ | Me | H | |
| 15 | I | CHF₂ | Me | H | |
| 16 | H | CHF₂ | Et | H | |
| 17 | F | CHF₂ | Et | H | |
| 18 | Cl | CHF₂ | Et | H | |
| 19 | Br | CHF₂ | Et | H | |
| 20 | I | CHF₂ | Et | H | |
| 21 | H | CF₃ | Me | F | 6.19 (s, Pyrazolyl-H) |
| 22 | F | CF₃ | Me | F | 6.22 (s, Pyrazolyl-H) |
| 23 | Cl | CF₃ | Me | F | 6.21 (s, Pyrazolyl-H) |
| 24 | Br | CF₃ | Me | F | 6.21 (s, Pyrazolyl-H) |
| 25 | I | CF₃ | Me | F | 6.20 (s, Pyrazolyl-H) |
| 26 | H | CF₃ | Et | F | |
| 27 | F | CF₃ | Et | F | |
| 28 | Cl | CF₃ | Et | F | |
| 29 | Br | CF₃ | Et | F | |
| 30 | I | CF₃ | Et | F | |
| 31 | H | CHF₂ | Me | F | |
| 32 | F | CHF₂ | Me | F | |
| 33 | Cl | CHF₂ | Me | F | |
| 34 | Br | CHF₂ | Me | F | |
| 35 | I | CHF₂ | Me | F | |
| 36 | H | CHF₂ | Et | F | |
| 37 | F | CHF₂ | Et | F | |
| 38 | Cl | CHF₂ | Et | F | |
| 39 | Br | CHF₂ | Et | F | |
| 40 | I | CHF₂ | Et | F | |
| 41 | SMe | CF₃ | Me | H | 6.18 |
| 42 | SMe | CF₃ | Me | F | 6.18 |
| 43 | SMe | CF₃ | Et | H | |
| 44 | SMe | CF₃ | Et | F | |
| 45 | SMe | CHF₂ | Me | H | |
| 46 | SMe | CHF₂ | Me | F | |
| 47 | SMe | CHF₂ | Et | H | |
| 48 | SMe | CHF₂ | Et | F | |

**Tabelle B: Erfindungsgemäße Verbindungen der allgemeinen Formel (I), worin die Substituenten und Symbole folgende Bedeutungen haben:**

| | | | | | |
|---|---|---|---|---|---|
| | | A = A2 | | | |
| 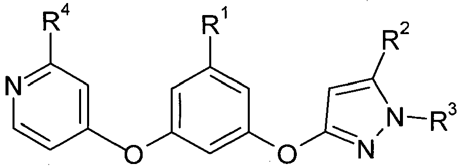 | | | | | |

| **Nr.** | **R¹** | **R²** | **R³** | **R⁴** | **Physikalische Daten: ¹H-NMR (CDCl₃)** |
|---|---|---|---|---|---|
| 1 | H | CF₃ | Me | OCF₂H | 6.21 (s, Pyrazolyl-H) |
| 2 | F | CF₃ | Me | OCF₂H | 6.23 (s, Pyrazolyl-H) |
| 3 | Cl | CF₃ | Me | OCF₂H | 6.23 (s, Pyrazolyl-H) |
| 4 | Br | CF₃ | Me | OCF₂H | 6.22 (s, Pyrazolyl-H) |
| 5 | I | CF₃ | Me | OCF₂H | 6.22 (s, Pyrazolyl-H) |
| 6 | H | CF₃ | Et | OCF₂H | |
| 7 | F | CF₃ | Et | OCF₂H | |
| 8 | Cl | CF₃ | Et | OCF₂H | |
| 9 | Br | CF₃ | Et | OCF₂H | |
| 10 | I | CF₃ | Et | OCF₂H | |
| 11 | H | CHF₂ | Me | OCF₂H | |
| 12 | F | CHF₂ | Me | OCF₂H | |
| 13 | Cl | CHF₂ | Me | OCF₂H | |
| 14 | Br | CHF₂ | Me | OCF₂H | |
| 15 | I | CHF₂ | Me | OCF₂H | |
| 16 | H | CHF₂ | Et | OCF₂H | |
| 17 | F | CHF₂ | Et | OCF₂H | |
| 18 | Cl | CHF₂ | Et | OCF₂H | |
| 19 | Br | CHF₂ | Et | OCF₂H | |
| 20 | I | CHF₂ | Et | OCF₂H | |
| 21 | H | CF₃ | Me | Cl | 6.19 (s, Pyrazolyl-H) |
| 22 | F | CF₃ | Me | Cl | |
| 23 | Cl | CF₃ | Me | Cl | |
| 24 | Br | CF₃ | Me | Cl | |
| 25 | I | CF₃ | Me | Cl | |
| 26 | H | CF₃ | Et | Cl | |
| 27 | F | CF₃ | Et | Cl | |
| 28 | Cl | CF₃ | Et | Cl | |
| 29 | Br | CF₃ | Et | Cl | |
| 30 | I | CF₃ | Et | Cl | |
| 31 | H | CHF₂ | Me | Cl | |
| 32 | F | CHF₂ | Me | Cl | |
| 33 | Cl | CHF₂ | Me | Cl | |
| 34 | Br | CHF₂ | Me | Cl | |
| 35 | I | CHF₂ | Me | Cl | |
| 36 | H | CHF₂ | Et | Cl | |
| 37 | F | CHF₂ | Et | Cl | |
| 38 | Cl | CHF₂ | Et | Cl | |
| 39 | Br | CHF₂ | Et | Cl | |
| 40 | I | CHF₂ | Et | Cl | |
| 41 | H | CF₃ | Me | CF₃ | 6.22 (s, Pyrazolyl-H) |
| 42 | F | CF₃ | Me | CF₃ | 6.23 (s, Pyrazolyl-H) |
| 43 | Cl | CF₃ | Me | CF₃ | 6.24 (s, Pyrazolyl-H) |
| 44 | Br | CF₃ | Me | CF₃ | 6.23 (s, Pyrazolyl-H) |
| 45 | I | CF₃ | Me | CF₃ | |
| 4.6 | H | CF₃ | Et | CF₃ | |
| 47 | F | CF₃ | Et | CF₃ | |
| 48 | Cl | CF₃ | Et | CF₃ | |
| 49 | Br | CF₃ | Et | CF₃ | |
| 50 | I | CF₃ | Et | CF₃ | |
| 51 | H | CHF₂ | Me | CF₃ | |
| 52 | F | CHF₂ | Me | CF₃ | |
| 53 | Cl | CHF₂ | Me | CF₃ | |
| 54 | Br | CHF₂ | Me | CF₃ | |
| 55 | I | CHF₂ | Me | CF₃ | |
| 56 | H | CHF₂ | Et | CF₃ | |
| 57 | F | CHF₂ | Et | CF₃ | |
| 58 | Cl | CHF₂ | Et | CF₃ | |
| 59 | Br | CHF₂ | Et | CF₃ | |
| 60 | I | CHF₂ | Et | CF₃ | |
| 61 | H | CF₃ | Me | CF₂H | |
| 62 | F | CF₃ | Me | CF₂H | |
| 63 | Cl | CF₃ | Me | CF₂H | |
| 64 | Br | CF₃ | Me | CF₂H | |
| 65 | I | CF₃ | Me | CF₂H | |
| 66 | H | CF₃ | Et | CF₂H | |
| 67 | F | CF₃ | Et | CF₂H | |
| 68 | Cl | CF₃ | Et | CF₂H | |
| 69 | Br | CF₃ | Et | CF₂H | |
| 70 | I | CF₃ | Et | CF₂H | |
| 71 | H | CHF₂ | Me | CF₂H | |
| 72 | F | CHF₂ | Me | CF₂H | |
| 73 | Cl | CHF₂ | Me | CF₂H | |
| 74 | Br | CHF₂ | Me | CF₂H | |
| 75 | I | CHF₂ | Me | CF₂H | |
| 76 | H | CHF₂ | Et | CF₂H | |
| 77 | F | CHF₂ | Et | CF₂H | |
| 78 | Cl | CHF₂ | Et | CF₂H | |
| 79 | Br | CHF₂ | Et | CF₂H | |
| 80 | I | CHF₂ | Et | CF₂H | |
| 81 | SMe | CF₃ | Me | Cl | |
| 82 | SMe | CF₃ | Me | OCF₂H | |
| 83 | SMe | CF₃ | Me | CF₃ | |
| 84 | SMe | CF₃ | Me | CF₂H | |
| 85 | SMe | CF₃ | Et | Cl | |
| 86 | SMe | CF₃ | Et | OCF₂H | |
| 87 | SMe | CF₃ | Et | CF₃ | |
| 88 | SMe | CF₃ | Et | CF₂H | |
| 89 | SMe | CHF₂ | Me | Cl | |
| 90 | SMe | CHF₂ | Me | OCF₂H | |
| 91 | SMe | CHF₂ | Me | CF₃ | |
| 92 | SMe | CHF₂ | Me | CF₂H | |
| 93 | SMe | CHF₂ | Et | Cl | |
| 94 | SMe | CHF₂ | Et | OCF₂H | |
| 95 | SMe | CHF₂ | Et | CF₃ | |
| 96 | SMe | CHF₂ | Et | CF₂H | |

**Tabelle C: Erfindungsgemäße Verbindungen der allgemeinen Formel (I), worin die Substituenten und Symbole folgende Bedeutungen haben:**

| | | | | |
|---|---|---|---|---|
| A = A3 | | R⁴ = CF₃ | | |
| 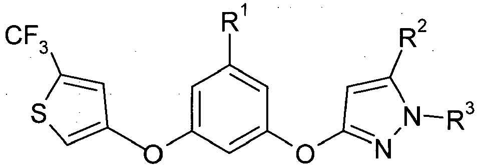 | | | | |

| **Nr.** | **R¹** | **R²** | **R³** | **Physikalische Daten: ¹H-NMR (CDCl₃)** |
|---|---|---|---|---|
| 1 | H | CF₃ | Me | |
| 2 | F | CF₃ | Me | |
| 3 | Cl | CF₃ | Me | |
| 4 | Br | CF₃ | Me | |
| 5 | I | CF₃ | Me | |
| 6 | H | CF₃ | Et | |
| 7 | F | CF₃ | Et | |
| 8 | Cl | CF₃ | Et | |
| 9 | Br | CF₃ | Et | |
| 10 | I | CF₃ | Et | |
| 11 | H | CHF₂ | Me | |
| 12 | F | CHF₂ | Me | |
| 13 | Cl | CHF₂ | Me | |
| 14 | Br | CHF₂ | Me | |
| 15 | I | CHF₂ | Me | |
| 16 | H | CHF₂ | Et | |
| 17 | F | CHF₂ | Et | |
| 18 | Cl | CHF₂ | Et | |
| 19 | Br | CHF₂ | Et | |
| 20 | I | CHF2 | Et | |
| 21 | SMe | CF₃ | Me | |
| 22 | SMe | CF₃ | Et | |
| 23 | SMe | CHF₂ | Me | |
| 24 | SMe | CHF₂ | Et | |

**Tabelle D: Erfindungsgemäße Verbindungen der allgemeinen Formel (I), worin die Substituenten und Symbole folgende Bedeutungen haben:**

| | | | | | |
|---|---|---|---|---|---|
| | A = A4 | | | R⁶ = Me | |
| 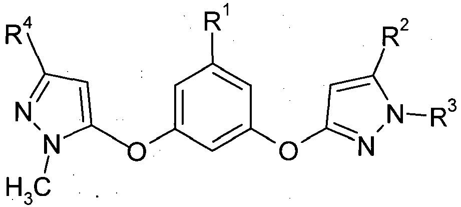 | | | | | |

| **Nr.** | **R¹** | **R²** | **R³** | **R⁴** | **Physikalische Daten: ¹H-NMR (CDCl₃)** |
|---|---|---|---|---|---|
| 1 | H | CF₃ | Me | CF₃ | 5.93 und 6.21, je (s, Pyrazolyl-H) |
| 2 | F | CF₃ | Me | CF₃ | 6.01 und 6.24, je (s, Pyrazolyl-H) |
| 3 | Cl | CF₃ | Me | CF₃ | 5.99 und 6.23, je (s, Pyrazolyl-H) |
| 4 | Br | CF₃ | Me | CF₃ | 5.98 und 6.22, je (s, Pyrazolyl-H) |
| 5 | I | CF₃ | Me | CF₃ | 5.97 und 6.22, je (s, Pyrazolyl-H) |
| 6 | H | CF₃ | Et | CF₃ | |
| 7 | F | CF₃ | Et | CF₃ | 6.03 und 6.22, je (s, Pyrazolyl-H) |
| 8 | Cl | CF₃ | Et | CF₃ | |
| 9 | Br | CF₃ | Et | CF₃ | |
| 10 | I | CF₃ | Et | CF₃ | |
| 11 | H | CHF₂ | Me | CF₃ | |
| 12 | F | CHF₂ | Me | CF₃ | |
| 13 | Cl | CHF₂ | Me | CF₃ | |
| 14 | Br | CHF₂ | Me | CF₃ | |
| 15 | I | CHF₂ | Me | CF₃ | |
| 16 | H | CHF₂ | Et | CF₃ | |
| 17 | F | CHF₂ | Et | CF₃ | |
| 18 | Cl | CHF₂ | Et | CF₃ | |
| 19 | Br | CHF₂ | Et | CF₃ | |
| 20 | I | CHF₂ | Et | CF₃ | |
| 21 | H | CF₃ | Me | CHF₂ | |
| 22 | F | CF₃ | Me | CHF₂ | |
| 23 | Cl | CF₃ | Me | CHF₂ | |
| 24 | Br | CF₃ | Me | CHF₂ | |
| 25 | I | CF₃ | Me | CHF₂ | |
| 26 | H | CF₃ | Et | CHF₂ | |
| 27 | F | CF₃ | Et | CHF₂ | |
| 28 | Cl | CF₃ | Et | CHF₂ | |
| 29 | Br | CF₃ | Et | CHF₂ | |
| 30 | I | CF₃ | Et | CHF₂ | |
| 31 | H | CHF₂ | Me | CHF₂ | |
| 32 | F | CHF₂ | Me | CHF₂ | |
| 33 | Cl | CHF₂ | Me | CHF₂ | |
| 34 | Br | CHF₂ | Me | CHF₂ | |
| 35 | I | CHF₂ | Me | CHF₂ | |
| 36 | H | CHF₂ | Et | CHF₂ | |
| 37 | F | CHF₂ | Et | CHF₂ | |
| 38 | Cl | CHF₂ | Et | CHF₂ | |
| 39 | Br | CHF₂ | Et | CHF₂ | |
| 40 | I | CHF₂ | Et | CHF₂ | |
| 41 | SMe | CF₃ | Me | CHF₂ | |
| 42 | SMe | CF₃ | Me | CF₃ | 5.94 und 6.20 |
| 43 | SMe | CF₃ | Et | CHF₂ | |
| 44 | SMe | CF₃ | Et | CF₃ | |
| 45 | SMe | CHF₂ | Me | CHF₂ | |
| 46 | SMe | CHF₂ | Me | CF₃ | |
| 47 | SMe | CHF₂ | Et | CHF₂ | |
| 48 | SMe | CHF₂ | Et | CF₃ | |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man 75 Gew.-Teile einer Verbindung der allgemeinen Formel(I),

| | |
|---|---|
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man 25 Gew.-Teile einer Verbindung der allgemeinen Formel (I),

| | |
|---|---|
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- und dikotylen Unkrautpflanzen werden in Papptöpfen in sandiger Lehmerde ausgelegt und mit Erde abgedeckt. Die in Form von benetzbaren Pulvern oder Emulsionskonzentraten formulierten Verbindungen werden dann als wäßrige Suspension bzw. Emulsion mit einer Wasseraufwandmenge von umgerechnet 600 bis 800 l/ha in einer Dosierung von umgerechnet 1000 g pro Hektar auf die Oberfläche der Abdeckerde appliziert. Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Nach 3 bis 4 Wochen Standzeit der Versuchspflanzen im Gewächshaus unter optimalen Wachstumsbedingungen wird die Wirkung der Verbindungen im Vergleich zu Verbindungen, die im Stand der Technik offenbart sind, bonitiert. Wie die Ergebnisse der Vergleichstabelle 1 zeigen, weisen die ausgewählten erfindungsgemäßen Verbindungen dabei eine besssere herbizide Wirksamkeit gegen ein breites Spektrum wirtschaftlich wichtiger mono- und dikotyler Schadpflanzen auf als die im Stand der Technik offenbarten.

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha in einer Dosierung von umgerechnet 1000g pro Hektar auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen im Vergleich zu Verbindungen, die im Stand der Technik offenbart sind, bonitiert. Wie die Ergebnisse der Vergleichstabellen 2 bis 4 zeigen, weisen die ausgewählten erfindungsgemäßen Verbindungen dabei eine besssere herbizide Wirksamkeit gegen ein breites Spektrum wirtschaftlich wichtiger mono- und dikotyler Schadpflanzen auf als die im Stand der Technik offenbarten.

### 3. Kulturpflanzenverträglichkeit

In weiteren Versuchen im Gewächshaus werden Samen von Gerste und mono- und dikotyler Schadpflanzen in sandigem Lehmboden ausgelegt, mit Erde abgedeckt und im Gewächshaus aufgestellt, bis die Pflanzen zwei bis drei echte Blätter entwickelt haben. Die Behandlung mit den erfindungsgemäßen Verbindungen der Formel (I) und im Vergleich dazu mit den im Stand der Technik offenbarten erfolgt dann wie oben unter Punkt 1 beschrieben. Vier bis fünf Wochen nach der Applikation und Standzeit im Gewächshaus wird mittels optischer Bonitur festgestellt, daß die erfindungsgemäßen Verbindungen im Gegensatz zu den im Stand der Technik offenbarten Verbindungen die Kulturpflanze selbst bei höheren Wirkstoffdosierungen ungeschädigt lassen.

**Erfindungsgemäße Verbindungen**

| Nr. | Struktur | |
|---|---|---|
| E1 | 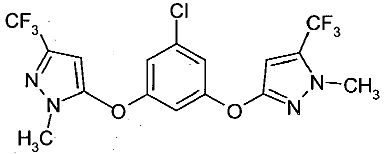 | (Nr. 3 aus Tabelle D) |
| E3 | 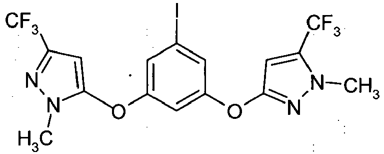 | (Nr. 5 aus Tabelle D) |
| E4 | 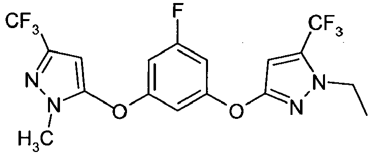 | (Nr. 7 aus Tabelle D) |

**Aus dem Stand der Technik (WO 2003/051846) bekannte Verbindungen:**

| Nr. | Struktur | |
|---|---|---|
| S1 | 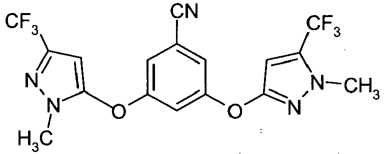 | (Nr. 75a aus WO 2003/051846) |
| S2 | 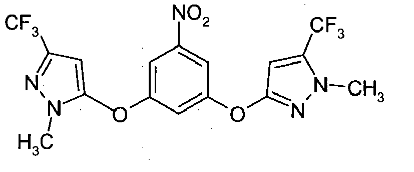 | (Nr. 396a aus WO 2003/051846) |

**Die in folgenden Vergleichstabellen verwendeten Abkürzungen bedeuten:**

| | | | |
|---|---|---|---|
| AMARE | Amaranthus retroflexus | AVESA | Avena fatua |
| LOLMU | Lolium multiflorum | SETVI | Setaria viridis |
| SINAL | Sinapis arvensis | STEME | Stellaria media |

**Vergleichstabelle 1, Vorauflauf**

| **Verbindung Nr.** | **Dosierung [g a.i./ha]** | **Schädigung der Schadpflanzen in %** | | |
|---|---|---|---|---|
| | | **AVESA** | **SINAL** | **STEME** |
| E1 | 1000 | 90% | 100% | 100% |
| E4 | 1000 | 100% | 100% | 100% |
| *S2* | *1000* | *70%* | *70%* | *90%* |

**Vergleichstabelle 2, Nachauflauf**

| **Verbindung Nr.** | **Dosierung [g a.i./ha]** | **Schädigung der Schadpflanzen in %** | | | | |
|---|---|---|---|---|---|---|
| | | **AMARE** | **AVESA** | **LOLMU** | **SETVI** | **SINAL** |
| E1 | 1000 | 100% | 90% | 100% | 90% | 90% |
| *S1* | *1000* | *80%* | *80%* | *80%* | *80%* | *80%* |

**Vergleichstabelle 3, Nachauflauf**

| **Verbindung Nr.** | **Dosierung [g a.i./ha]** | **Schädigung der Schadpflanzen in %** | | | |
|---|---|---|---|---|---|
| | | **AVESA** | **LOLMU** | **SETVI** | **STEME** |
| E3 | 1000 | 80% | 90% | 90% | 90% |
| *S2* | *1000* | *60%* | *60%* | *70%* | *80%* |

**Vergleichstabelle 4, Nachauflauf**

| **Verbindung Nr.** | **Dosierung [g a.i./ha]** | **Schädigung der Schadpflanzen in %** | | | |
|---|---|---|---|---|---|
| | | **AMARE** | **SETVI** | **SINAL** | **STEME** |
| E4 | 1000 | 100% | 100% | 100% | 100% |
| *S1* | *1000* | *80%* | *80%* | *80%* | *80%* |
| *S2* | *1000* | *70%* | *90%* | *90%* | *80%* |

## Patentansprüche

1. Pyrazolyloxyphenylderivate der Formel (I) und deren Salze 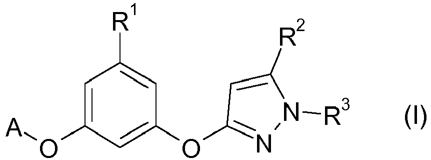 worin die Substituenten und Indices folgende Bedeutungen haben:
R¹ bedeutet Brom, Chlor, Fluor oder Iod;
R² bedeutet Trifluormethyl, Difluormethyl oder Chlordifluormethyl;
R³ bedeutet Methyl oder Ethyl;
A bedeutet einen Rest aus der Gruppe umfassend die Reste A1 bis A4 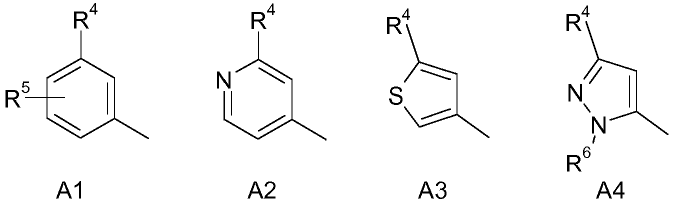
R⁴ bedeutet Fluormethyl, Difluormethyl, Trifluormethyl, Fluormethoxy, Difluormethoxy, Trifluormethoxy, Chlor oder Cyano;
R⁵ bedeutet Wasserstoff, (C₁-C₈)-Alkyl, Brom, Chlor, Fluor, Iod oder Cyano, und
R⁶ bedeutet (C₁-C₈)-Alkyl.

2. Pyrazolyloxyphenylderivate nach Anspruch 1, worin
R¹ bedeutet Brom, Chlor, Fluor oder Iod;
R² bedeutet Trifluormethyl oder Difluormethyl;
R³ bedeutet Methyl oder Ethyl;
A bedeutet einen Rest aus der Gruppe umfassend die Reste A1 bis A4;
R⁴ bedeutet Difluormethyl, Trifluormethyl, Difluormethoxy, Trifluormethoxy, Chlor oder Cyano;
R⁵ bedeutet Wasserstoff, Fluor oder Chlor;
R⁶ bedeutet Methyl oder Ethyl.

3. Pyrazolyloxyphenylderivate nach Anspruch 1 oder 2, worin
R¹ bedeutet Brom, Chlor, Fluor oder Iod,
R² bedeutet Trifluormethyl oder Difluormethyl;
R³ bedeutet Methyl oder Ethyl;
A bedeutet einen Rest aus der Gruppe umfassend die Reste A1 bis A4;
R⁴ bedeutet Difluormethyl, Trifluormethyl, Difluormethoxy, Trifluormethoxy, Chlor oder Cyano;
R⁵ bedeutet Wasserstoff oder Fluor;
R⁶ bedeutet Methyl.

4. Pyrazolyloxyphenylderivate nach einem der Ansprüche 1 bis 3, worin R¹ bedeutet Brom, Chlor, Fluor oder Iod;
R² bedeutet Trifluormethyl oder Difluormethyl;
R³ bedeutet Methyl oder Ethyl;
A bedeutet den Rest A1;
R⁴ bedeutet Difluormethyl, Trifluormethyl, Difluormethoxy, Trifluormethoxy, Chlor oder Cyano;
R⁵ bedeutet Wasserstoff oder Fluor.

5. Pyrazolyloxyphenylderivate nach einem der Ansprüche 1 bis 3, worin R¹ bedeutet Brom, Chlor, Fluor oder Iod,
R² bedeutet Trifluormethyl oder Difluormethyl;
R³ bedeutet Methyl oder Ethyl;
A bedeutet den Rest A2;
R⁴ bedeutet Difluormethyl, Trifluormethyl, Difluormethoxy, Trifluormethoxy, Chlor oder Cyano.

6. Pyrazolyloxyphenylderivate nach einem der Ansprüche 1 bis 3, worin
R¹ bedeutet Brom, Chlor, Fluor oder Iod;
R² bedeutet Trifluormethyl oder Difluormethyl;
R³ bedeutet Methyl oder Ethyl;
A bedeutet den Rest A3;
R⁴ bedeutet Difluormethyl, Trifluormethyl, Difluormethoxy, Trifluormethoxy, Chlor oder Cyano.

7. Pyrazolyloxyphenylderivate nach einem der Ansprüche 1 bis 3, worin
R¹ bedeutet Brom, Chlor, Fluor oder Iod;
R² bedeutet Trifluormethyl oder Difluormethyl;
R³ bedeutet Methyl oder Ethyl;
A bedeutet den Rest A4;
R⁴ bedeutet Difluormethyl, Trifluormethyl, Difluormethoxy, Trifluormethoxy, Chlor oder Cyano;
R⁶ bedeutet Methyl.

8. Herbizide Mittel, **gekennzeichnet durch** einen herbizid wirksamen Gehalt an mindestens einer Verbindung der allgemeinen Formel (I) gemäß einem der Ansprüche 1 bis 7.

9. Herbizide Mittel nach Anspruch 8 in Mischung mit Formulierungshilfsmitteln.

10. Verfahren zur Bekämpfung unerwünschter Pflanzen, **dadurch gekennzeichnet, daß** man eine wirksame Menge mindestens einer Verbindung der allgemeinen Formel (I) nach einem der Ansprüche 1 bis 7 oder eines herbiziden Mittels nach Anspruch 8 oder 9 auf die Pflanzen oder auf den Ort des unerwünschten Pflanzenwachstums appliziert.

11. Verwendung von Verbindungen der allgemeinen Formel (I) nach einem der Ansprüche 1 bis 7 oder von herbiziden Mitteln nach Anspruch 8 oder 9 zur Bekämpfung unerwünschter Pflanzen.

12. Verwendung nach Anspruch 11, **dadurch gekennzeichnet, daß** die Verbindungen der allgemeinen Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. A pyrazolyloxyphenyl derivative of the formula (I) or a salt thereof 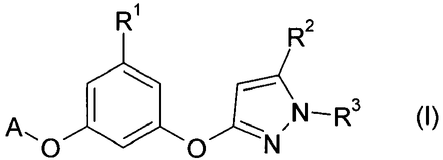 in which the substituents and indices are as defined below:
R¹ is bromine, chlorine, fluorine or iodine;
R² is trifluoromethyl, difluoromethyl or chlorodifluoromethyl;
R³ is methyl or ethyl;
A is a radical from the group consisting of the radicals A1 to A4 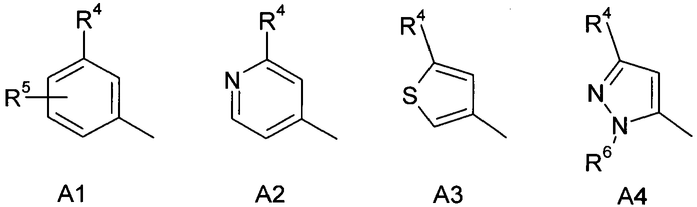
R⁴ is fluoromethyl, difluoromethyl, trifluoromethyl, fluoromethoxy, difluoromethoxy, trifluoromethoxy, chlorine or cyano;
R⁵ is hydrogen, (C₁-C₈)-alkyl, bromine, chlorine, fluorine, iodine or cyano, and
R⁶ is (C₁-C₈)-alkyl.

2. The pyrazolyloxyphenyl derivative as claimed in claim 1, in which
R¹ is bromine, chlorine, fluorine or iodine;
R² is trifluoromethyl or difluoromethyl;
R³ is methyl or ethyl;
A is a radical from the group consisting of the radicals A1 to A4;
R⁴ is difluoromethyl, trifluoromethyl, difluoromethoxy, trifluoromethoxy, chlorine or cyano;
R⁵ is hydrogen, fluorine or chorine;
R⁶ is methyl or ethyl.

3. The pyrazolyloxyphenyl derivative as claimed in claim 1 or 2, in which
R¹ is bromine, chlorine, fluorine or iodine;
R² is trifluoromethyl or difluoromethyl;
R³ is methyl or ethyl;
A is a radical from the group consisting of the radicals A1 to A4;
R⁴ is difluoromethyl, trifluoromethyl, difluoromethoxy, trifluoromethoxy, chlorine or cyano;
R⁵ is hydrogen or fluorine;
R⁶ is methyl.

4. The pyrazolyloxyphenyl derivative as claimed in any of claims 1 to 3, in which
R¹ is bromine, chlorine, fluorine or iodine;
R² is trifluoromethyl or difluoromethyl;
R³ is methyl or ethyl;
A is the radical A1;
R⁴ is difluoromethyl, trifluoromethyl, difluoromethoxy, trifluoromethoxy, chlorine or cyano;
R⁵ is hydrogen or fluorine.

5. The pyrazolyloxyphenyl derivative as claimed in one of claims 1 to 3, in which
R¹ is bromine, chlorine, fluorine or iodine;
R² is trifluoromethyl or difluoromethyl;
R³ is methyl or ethyl;
A is the radical A2;
R⁴ is difluoromethyl, trifluoromethyl, difluoromethoxy, trifluoromethoxy, chlorine or cyano.

6. The pyrazolyloxyphenyl derivative as claimed in one of claims 1 to 3, in which
R¹ is bromine, chlorine, fluorine or iodine;
R² is trifluoromethyl or difluoromethyl;
R³ is methyl or ethyl;
A is the radical A3;
R⁴ is difluoromethyl, trifluoromethyl, difluoromethoxy, trifluoromethoxy, chlorine or cyano.

7. The pyrazolyloxyphenyl derivative as claimed in one of claims 1 to 3, in which
R¹ is bromine, chlorine, fluorine or iodine;
R² is trifluoromethyl or difluoromethyl;
R³ is methyl or ethyl;
A is the radical A4;
R⁴ is difluoromethyl, trifluoromethyl, difluoromethoxy, trifluoromethoxy, chlorine or cyano;
R⁶ represents methyl.

8. A herbicidal composition which comprises a herbicidally effective amount of at least one compound of the formula (I) as claimed in one of claims 1 to 7.

9. The herbicidal composition as claimed in claim 8 in a mixture with formulation auxiliaries.

10. A method for controlling unwanted plants which comprises applying an effective amount of at least one compound of the formula (I) as claimed in one of claims 1 to 7 or of a herbicidal composition as claimed in claim 8 or 9 to the plants or the location of the unwanted vegetation.

11. The use of compounds of the formula (I) as claimed in one of claims 1 to 7 or of herbicidal compositions as claimed in claim 8 or 9 for controlling unwanted plants.

12. The use as claimed in claim 11, wherein the compounds of the formula (I) are used for controlling unwanted plants in crops of useful plants.

13. The use as claimed in claim 12, wherein the useful plants are transgenic useful plants.

## Revendications

1. Dérivés de pyrazolyloxyphényle de formule (I) et leurs sels 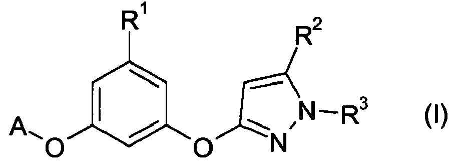 dans laquelle les substituants et les indices présentent les significations suivantes :
R¹ signifie brome, chlore, fluor ou iode ;
R² signifie trifluorométhyle, difluorométhyle ou chlorodifluorométhyle ;
R³ signifie méthyle ou éthyle ;
A signifie un radical du groupe comprenant les radicaux A1 à A4 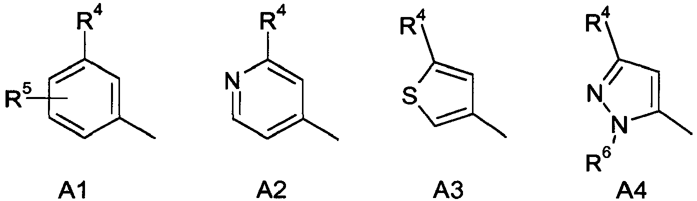
R⁴ signifie fluorométhyle, difluorométhyle, trifluorométhyle, fluorométhoxy, difluorométhoxy, trifluorométhoxy, chlore ou cyano ;
R⁵ signifie hydrogène, (C₁-C₈)-alkyle, brome, chlore, fluor, iode ou cyano et
R⁶ signifie (C₁-C₈)-alkyle.

2. Dérivés de pyrazolyloxyphényle selon la revendication 1, dans lesquels
R¹ signifie brome, chlore, fluor ou iode ;
R² signifie trifluorométhyle ou difluorométhyle ;
R³ signifie méthyle ou éthyle ;
A signifie un radical du groupe comprenant les radicaux A1 à A4 ;
R⁴ signifie difluorométhyle, trifluorométhyle, difluorométhoxy, trifluorométhoxy, chlore ou cyano ;
R⁵ signifie hydrogène, fluor ou chlore ;
R⁶ signifie méthyle ou éthyle.

3. Dérivés de pyrazolyloxyphényle selon la revendication 1 ou 2, dans lesquels
R¹ signifie brome, chlore, fluor ou iode ;
R² signifie trifluorométhyle ou difluorométhyle ;
R³ signifie méthyle ou éthyle ;
A signifie un radical du groupe comprenant les radicaux A1 à A4 ;
R⁴ signifie difluorométhyle, trifluorométhyle, difluorométhoxy, trifluorométhoxy, chlore ou cyano ;
R⁵ signifie hydrogène ou fluor ;
R⁶ signifie méthyle.

4. Dérivés de pyrazolyloxyphényle selon l'une quelconque des revendications 1 à 3, dans lesquels
R¹ signifie brome, chlore, fluor ou iode ;
R² signifie trifluorométhyle ou difluorométhyle ;
R³ signifie méthyle ou éthyle ;
A signifie le radical A1 ;
R⁴ signifie difluorométhyle, trifluorométhyle, difluorométhoxy, trifluorométhoxy, chlore ou cyano ;
R⁵ signifie hydrogène ou fluor.

5. Dérivés de pyrazolyloxyphényle selon l'une quelconque des revendications 1 à 3, dans lesquels R¹ signifie brome, chlore, fluor ou iode ;
R² signifie trifluorométhyle ou difluorométhyle ;
R³ signifie méthyle ou éthyle ;
A signifie le radical A2 ;
R⁴ signifie difluorométhyle, trifluorométhyle, difluorométhoxy, trifluorométhoxy, chlore ou cyano.

6. Dérivés de pyrazolyloxyphényle selon l'une quelconque des revendications 1 à 3, dans lesquels
R¹ signifie brome, chlore, fluor ou iode ;
R² signifie trifluorométhyle ou difluorométhyle ;
R³ signifie méthyle ou éthyle ;
A signifie le radical A3 ;
R⁴ signifie difluorométhyle, trifluorométhyle, difluorométhoxy, trifluorométhoxy, chlore ou cyano.

7. Dérivés de pyrazolyloxyphényle selon l'une quelconque des revendications 1 à 3, dans lesquels
R¹ signifie brome, chlore, fluor ou iode ;
R² signifie trifluorométhyle ou difluorométhyle ;
R³ signifie méthyle ou éthyle ;
A signifie le radical A4 ;
R⁴ signifie difluorométhyle, trifluorométhyle, difluorométhoxy, trifluorométhoxy, chlore ou cyano ;
R⁶ signifie méthyle.

8. Agents herbicides, **caractérisés par** une teneur active en tant qu'herbicide en au moins un composé de formule générale (I) selon l'une quelconque des revendications 1 à 7.

9. Agents herbicides selon la revendication 8 en mélange avec des adjuvants de formulation.

10. Procédé pour lutter contre des plantes non souhaitées, **caractérisé en ce qu'**on applique une quantité active d'au moins un composé de formule générale (I) selon l'une quelconque des revendications 1 à 7 ou d'un agent herbicide selon la revendication 8 à 9 sur les plantes ou sur le lieu de la croissance des plantes non souhaitées.

11. Utilisation de composés de formule générale (I) selon l'une quelconque des revendications 1 à 7 ou d'agents herbicides selon la revendication 8 ou 9 pour lutter contre des plantes non souhaitées.

12. Utilisation selon la revendication 11, **caractérisée en ce que** les composés de formule générale (I) sont utilisés pour lutter contre des plantes non souhaitées dans les cultures de plantes utiles.

13. Utilisation selon la revendication 12, **caractérisée en ce que** les plantes utiles sont des plantes utiles transgéniques.
